# EUROPÄISCHE PATENTSCHRIFT

(11) **EP 1 420 685 B1**
(45) Veröffentlichungstag und Bekanntmachung des Hinweises auf die Patenterteilung: **26.08.2015**
(21) Anmeldenummer: 02758486.1
(22) Anmeldetag: 27.08.2002
(51) Int. Cl.: A61B 5/00

(54) **VERFAHREN, VORRICHTUNG UND SYSTEM ZUR MESSUNG UND ÜBERTRAGUNG VON KÖRPERFUNKTIONSWERTEN**
METHOD, DEVICE AND SYSTEM FOR MEASURING AND TRANSMITTING FUNCTIONAL VALUES OF THE BODY
PROCEDE, DISPOSITIF ET SYSTEME POUR LA MESURE ET LA TRANSMISSION DE VALEURS FONCTIONNELLES DU CORPS

(30) Priorität: 27.08.2001 DE 10140968
(43) Veröffentlichungstag der Anmeldung: 26.05.2004
(73) Patentinhaber: VITA PHONE GmbH, 68161 Mannheim (DE)
(72) Erfinder: LAMSTER, Jörg, 99192 Neudietendorf (DE)
(74) Vertreter: Von Rohr Patentanwälte Partnerschaft mbB
(86) Internationale Anmeldenummer: PCT/EP2002/009551
(87) Internationale Veröffentlichungsnummer: WO 2003/017832

(56) Entgegenhaltungen:
- EP-A- 1 101 437
- DE-A- 10 006 598
- DE-A- 19 848 229
- US-A- 5 772 586
- US-A- 5 967 975

## Beschreibung

Die Erfindung betrifft ein Verfahren zur Messung und Übertragung von Körperfunktionswerten eines Patienten, insbesondere von Blutzuckerwerten, nach dem Oberbegriff des Anspruchs 1.

Aus der DE 100 06 598 A1 ist ein Verfahren zur Messung und Übertragung von Funktionswerten von Körperfunktionen der eingangs genannten Art bekannt. Dort ist eine Anordnung mit einem Meßwertaufnehmer offenbart, welcher beispielsweise Blutzuckerwerte mißt. Der Meßwertaufnehmer weist einen Sender zur drahtlosen Übertragung von Meßdaten auf. Die Meßdaten werden als Datensignale vom Sender zu einem Handy übertragen, von dem aus eine Datenübertragung zu einer Datenbank mit einer Auswerteeinrichtung möglich ist.

Im Stand der Technik werden die gemessenen Körperfunktionswerte üblicherweise als Datensignale über ein Funknetz übertragen und nach dem Empfang auf einer Empfangseinrichtung des Empfängers an eine Auswerteeinrichtung weitergeleitet. In der Auswerteeinrichtung des Empfängers werden die empfangenen Datensignale ausgewertet. Ziel der Auswertung ist dabei, die Daten in eine verständliche Nachricht zu transformieren, welche der Empfänger durch Lesen, Hören oder Betrachten verstehen und bewerten kann.

Aus der medizinischen Analytik sind darüber hinaus Verfahren zur Messung und drahtlosen Übertragung der Funktionswerte von Körperfunktionen bekannt, bei denen das Meßgerät unmittelbar am Körper oder in unmittelbarer Nähe zum Körper angeordnet ist und wobei meßwertspezifische Informationen von dem Meßgerät drahtlos an eine Auswertungseinrichtung bzw. eine Speichereinrichtung übertragen werden. Diese Verfahren lassen nur eine begrenzte räumliche Distanz zwischen dem Meßgerät und der Auswertungseinrichtung bzw. der Speichereinrichtung zu, da in der Regel die Leistungsfähigkeit des Funknetzes begrenzt ist. Dies führt dazu, daß die Mobilität des Patienten während der Messung stark eingeschränkt ist.

Bei den aus der Praxis bekannten Verfahren zur Messung und drahtlosen Übertragung der Funktionswerte von Körperfunktionen ist es zudem so, daß unterschiedliche Meßgeräte und unterschiedliche Auswertungseinrichtungen bzw. Speichereinrichtungen nicht miteinander kompatibel sind. Die Übertragung meßwertspezifischer Informationen ist somit beschränkt auf unmittelbar zusammenwirkende Meßgeräte und Auswertungseinrichtungen bzw. Speichereinrichtungen.

Aufgabe der vorliegenden Erfindung ist es, ein System der eingangs genannten Art zur Verfügung zu stellen, das eine möglichst schnelle und einfache Auswertung der gemessenen Körperfunktionswerte ermöglicht, um eine einfache und schnelle Therapierung des Patienten im Falle von Komplikationen zu gewährleisten.

Die vorgenannte Aufgabe ist bei einem Verfahren der eingangs genannten Art durch die Merkmale des Anspruchs 1 gelöst. Bevorzugte Ausführungsformen sind Gegenstand der Unteransprüche.

Erfindungsgemäß wird ein neuer Weg zur Übertragung gemessener Körperfunktionswerte vorgeschlagen, wobei die meßwertspezifische Information direkt in Form einer elektronischen Nachricht an den Empfänger, wobei es sich beispielsweise um einen Arzt oder ein medizinisches Service-Center handelt, übertragen wird. Der Begriff "Nachricht" weist darauf hin, daß die übertragenen Körperfunktionswerte schon beim Empfang auf dem Empfangsgerät des Empfängers als Nachricht ausgegeben werden können, ohne zuvor einer Auswerteeinrichtung zugeführt werden zu müssen. Der Empfänger kann die übertragenen meßwertspezifischen Informationen, nämlich die gemessenen Körperfunktionswerte, direkt und unverzüglich nach dem Empfang allein durch Hören und/oder durch Lesen und/oder durch Betrachten der elektronischen Nachricht, bei der es sich beispielsweise um eine SMS oder dgl. handelt, ohne weitere Benutzereingriffe erfassen bzw. "verstehen". Dies erleichtert die Auswertung erheblich und bietet die Möglichkeit, daß Rettungsmaßnahmen unverzüglich eingeleitet werden können.

Von erheblichem Vorteil ist dabei im übrigen, daß standardisierte elektronische Nachrichten übertragen werden, welche nahezu von jedem mit den Mobilfunknetz kommunizierenden Empfangsgerät üblicher Bauart kompatibel = "lesbar" sind. Der Begriff "standardisiert" bezieht sich dabei auf den Typ der elektronischen Nachricht. Eine standardisierte Nachricht ist beispielsweise eine SMS. Wird die meßwertspezifische Information als SMS übertragen, ist es grundsätzlich möglich, diese SMS an nahezu jedes beliebige Mobilfunkgerät bzw. Handy zu senden, das mit dem Mobilfunknetz verbunden ist. Ist das Empfangsgerät des Empfängers ausgeschaltet oder hat keinen Empfang, womit jedenfalls bei einem stationären Service-Center nicht zu rechnen ist, wird die elektronische Nachricht vom Netzbetreiber des Telekommunikationsdienstes für eine gewisse Zeitdauer aufbewahrt und zugestellt, sobald das Empfangsgerät wieder im Netz angemeldet ist. Auf diese Weise gehen keine Nachrichten verloren, was letztlich zu der hohen Sicherheit des erfindungsgemäßen Verfahrens beiträgt.

Die Übertragung als elektronische Nachricht in einem Standardprotokoll bietet sich neben der Einfachheit und Sicherheit der Datenübertragung nicht zuletzt auch aufgrund der vielfältigen Einsatzmöglichkeiten und der hohen Marktdurchdringung von Standardprotokollen im Mobilfunkverkehr an. Zudem ist es möglich, die stete Weiterentwicklung der Telekommunikationsdienste unmittelbar bei der Übertragung meßwertspezifischer Informationen zu nutzen. Auch ist es von Vorteil, daß Standardprotokolle die Übertragung "lesbarer" elektronischer Nachrichten ermöglichen, so daß eine medizinische Intervention unmittelbar nach dem Eingang der übertragenen meßwertspezifischen Information auf einer Empfangseinrichtung erfolgen kann. So ist es z.B. vorstellbar, daß die meßwertspezifische Information als Meßwert an eine mobile Sende- und Empfangseinrichtung des Arztes direkt übertragen wird. Es ist sogar möglich, daß ein gesamtes Meßprogramm von unterschiedlichen körperfunktionsbeschreibenden Meßwerten gleichzeitig übertragen wird.

Erfindungsgemäß wird vorzugsweise vorgesehen, die meßwertspezifische Information als Kurznachricht im Format eines Short-Message-Service (SMS) in ein nach dem GSM-Standard arbeitendes Mobilfunknetz zu übertragen. SMS-Nachrichten sind derzeit auf eine maximale Länge von 160 Zeichen begrenzt. Daher werden leistungsfähigere Nachfolger wie beispielsweise EMS (Enhanced Messaging) und MMS (Multimedia Messaging) zukünftig die Möglichkeiten der elektronischen Nachrichtenübertragung deutlich verbessern. Derzeit bietet sich jedoch die Übertragung als SMS aufgrund der Einfachheit und Sicherheit der Datenübertragung und nicht zuletzt aufgrund der hohen Marktdurchdringung von SMS-Nachrichten an. Ein zukünftiger Einsatz des erfindungsgemäßen Verfahrens kann jedoch auch vorsehen, daß aufgrund der höheren Datenübertragungsraten die elektronischen Nachrichten in Mobilfunknetzen übertragen werden, die nach anderen Standards arbeiten, beispielsweise in einem nach dem GPRS- und/oder einem nach dem UMTS-Standard arbeitenden Mobilfunksystem, und dort beispielsweise als MMS übertragen werden. Vor allem die hohen Datenübertragungsraten der zukünftigen Mobilfunkstandards eröffnen breit gefächerte Einsatzmöglichkeiten des erfindungsgemäßen Verfahrens und erlauben die Steigerung der übertragenden Datenmengen. Auch die Einbindung der Mobilfunktechnologie in das World Wide Web ist in diesem Zusammenhang von Interesse, wobei die meßwertspezifischen Informationen weltweit abrufbar sind und in beliebiger Art und Weise ausgewertet und gespeichert werden können. Hinterlegte Patientendaten können bei entsprechender Legitimation und Zugangserlaubnis online abgefragt werden. Dies erlaubt eine zeitnahe Reaktion auf kritische Körperfunktionszustände. Zudem werden Arztbesuche nicht mehr in jedem Fall erforderlich.

Es ist auch grundsätzlich auch möglich, daß das Meßgerät die meßwertspezifische Information in einem Standardprotokoll eines Telekommunikationsdienstes an die mobile Sende- und Empfangsreinrichtung überträgt. Werden die meßwertspezifischen Informationen alle in demselben Standardprotokoll eines Telekommunikationsdienstes übertragen, trägt dieses zur Vereinfachung der Informationsübertragung bei. Vorzugsweise wird die meßwertspezifische Information vom Meßgerät drahtlos über eine Blue-Tooth-Funkverbindung an die mobile Sende- und Empfangseinrichtung übertragen, wobei das Meßgerät und die mobile Sende- und Empfangseinrichtung ein Verhältnis von "Master" zu "Slave" haben. Der Blue-Tooth-Standard zeichnet sich durch eine sehr hohe Übertragungsrate aus. Es ist jedoch selbstverständlich auch denkbar, daß die Datenübertragung vom Meßgerät an die mobile Sende- und Empfangseinrichtung per Infrarot oder per Ultraschall erfolgen kann.

Als mobile Sende- und Empfangseinheit kann erfindungsgemäß vorzugsweise ein Mobiltelefon eingesetzt werden. Damit kann beispielsweise der Patient in einem kritischen Zustand unmittelbar mit dem Arzt und/oder einem Ansprechpartner beispielsweise eines medizinischen Callcenters telefonieren. Zudem ist es erfindungsgemäß möglich, daß bei Unter- bzw. Überschreiten definierter Sollwerte oder bei Generierung einer Therapieempfehlung zusätzlich zu der meßwertspezifischen Information bzw. der Therapieempfehlung eine Sprachmitteilung, beispielsweise als Alarmierungs- oder Warnanruf, übertragen wird.

Es ist darüber hinaus möglich, daß die mobile Sende- und Empfangseinrichtung bei Unter- bzw. Überschreiten definierter Sollwerte automatisch eine Telefonverbindung zwischen der Auswertungseinrichtung und/oder einem Arzt oder einem Rettungsdienst aufbaut, worauf nachfolgend noch näher eingegangen wird. Dadurch können Notfälle schneller als bisher therapiert werden, zudem sinkt der organisatorische Aufwand der Patientenbetreuung. Selbstverständlich ist es jedoch auch vorstellbar, beliebige mobile Sende- und Empfangseinrichtungen einzusetzen, die miteinander kommunizieren. Die Auswahl des Mobilfunknetzes unterliegt lediglich der Forderung, daß die Übertragung der meßwertspezifischen Information von der mobilen Sende- und Empfangseinheit in das jeweilige Mobilfunknetz gesichert sein muß.

Das Meßgerät selbst weist wenigstens eine Meßeinrichtung zur Erfassung des Meßwertes auf. Der erfaßte Meßwert, bei dem es sich in der Regel um physikalische und/oder chemische Parameter bzw. Datensignale handelt, wird über wenigstens einen Meßwertwandler in die meßwertspezifische Information transformiert. In diesem Fall kann es sich bei der meßwertspezifischen Information folglich um einen dem Meßwert entsprechenden Zahlenwert handeln. Darüber hinaus kann es sich bei der meßwertspezifischen Information auch um eine in Abhängigkeit vom Meßwert generierte Therapieempfehlung handeln. Über eine integrierte Sendeeinrichtung des Meßgerätes wird die meßwertspezifische Information anschließend an die mobile Sende- und Empfangseinrichtung übertragen. Um Informationen zu empfangen, ist es selbstverständlich auch möglich, eine Empfangseinrichtung in das Meßgerät zu integrieren. Die verschiedenen Einrichtungen des Meßgerätes sind in das Meßgerät integriert, wodurch eine hohe Kompaktheit des Meßgerätes und damit ein möglichst geringer Behinderungsgrad des Patienten, der dieses Meßgerät dauerhaft benutzt, gewährleistet wird. Es ist dabei ausreichend, die Leistungsfähigkeit der in dem Meßgerät integrierten Sendeeinrichtung so zu begrenzen, daß lediglich eine Verbindung zwischen der in dem Meßgerät integrierten Sendeeinrichtung des Meßgerätes und der mobilen Sende- und Empfangseinrichtung besteht. Dadurch kann die Baugröße und das Gewicht des Meßgerätes minimiert werden.

Des weiteren ist es bei dem erfindungsgemäßen Verfahren vorgesehen, die meßwertspezifische Information von der mobilen Sende- und Empfangseinrichtung an wenigstens eine Auswertungseinrichtung über das Mobilfunknetz zu übertragen. Vor allem bei komplexen Datensätzen und bei Funktionswerten von Körperfunktionen, die einer aufwendigen Analyse zugeführt werden müssen, bietet es sich an, eine Auswertungseinrichtung vorzusehen.

Auf der Grundlage des Meßwertes kann eine Therapieempfehlung zur Therapierung eines Fehlverhaltens der Körperfunktion generiert werden. Bei der Therapieempfehlung kann es sich beispielsweise um eine Textnachricht handeln, aus der ein Warnhinweis und/oder der Meßwert als Zahlenwert hervorgeht bzw. in welche der Meßwert als Zahlenwert integriert ist. Selbstverständlich kann die Genierung einer Therapieempfehlung auch unmittelbar durch den behandelnden Arzt bzw. in der Auswertungseinrichtung und/oder in der mobilen Sende- und Empfangseinrichtung oder im Meßgerät selbst erfolgen.

Als meßwertspezifische Information kann entweder ein dem Meßwert entsprechender Zahlenwert und/oder die Therapieempfehlung übertragen werden. Wesentlich ist, daß die meßwertspezifische Information als lesbare und/oder hörbare elektronische Nachricht übertragen wird. Enthält die meßwertspezifische Information sowohl den gemessenen Meßwert als Zahlenwert als auch die daraus abzuleitende Therapieempfehlung, kann dem Empfänger durch die elektronische Nachricht ein vergleichsweise breiter Informationsgehalt mitgeteilt werden, der zur Anleitung des Empfängers dienen kann. Beispielsweise ist es möglich, daß der Arzt oder das medizinische Service-Center bzw. die Auswertungseinrichtung als Empfänger eine elektronische Nachricht in Form einer SMS erhält mit dem Text "Kein Notfall, der Patient XY hat einen Blutzuckerwert von 75 mg/dl", wobei in diesem Beispiel der Meßwert als Zahl ("75") in die Therapieempfehlung ("Kein Notfall, der Patient XY hat einen Blutzuckerwert von ... mg/dl") integriert ist. Nach dem Eingang der Nachricht auf seinem Empfangsgerät kann der Empfänger der SMS schon beim Lesen der Nachricht darüber entscheiden, ob Therapiemaßnahmen eingeleitet werden müssen oder nicht.

Zudem kann der Empfänger dem Patienten unmittelbar antworten, vorzugsweise ebenfalls in Form einer elektronischen Nachricht, beispielsweise per SMS. Dabei ist von Vorteil, daß bei Erhalt einer SMS stets die Telefonnummer des Absenders mitübertragen wird, so daß der Empfänger stets weiß, wer die SMS abgesendet hat bzw. von wem die gemessenen Körperfunktionswerte stammen.

Grundsätzlich ist es natürlich auch möglich, daß zusammen mit der Therapieempfehlung weitere fachspezifische Informationen, wie beispielsweise Patientendaten oder der Krankheitsverlauf des Patienten zusammen mit dem einem Meßwert entsprechenden Zahlenwert übertragen werden. Darüber hinaus kann es auch von Vorteil sein, daß als meßwertspezifische Information ausschließlich die Therapieempfehlung übertragen wird, wobei die Therapieempfehlung vorzugsweise derart gestaltet sein sollte, daß auch ein nicht als Mediziner ausgebildeter Empfänger aus dem Bedeutungsinhalt der empfangenen Nachricht unmittelbar schließen kann, ob ein Notfall vorliegt oder nicht. Beispielsweise kann es sich bei der Therapieempfehlung in diesem Fall lediglich um einen Warnhinweis handeln, der den Empfänger auffordert, weitere Schritte einzuleiten, insbesondere einen Arzt zu verständigen. Dabei ist es von Vorteil, daß die zu übertragende meßwertspezifische Information als elektronische Nachricht auf der mobilen Sende- und Empfangseinrichtung angezeigt wird. Dadurch kann der Patient, dessen Körperfunktionswerte gemessen worden sind, unmittelbar auf eine bevorstehende Verschlechterung seines Gesundheitszustandes aufmerksam gemacht werden.

Es ist von weiter Vorteil, daß die Therapieempfehlung auf der Grundlage eines Sollwert-Istwert-Vergleichs der Meßwerte generiert wird. Dieser Sollwert-Istwert-Vergleich kann vorzugsweise mittels einer entsprechenden Software-Lösung durchgeführt werden und/oder manuell durch den Patienten und/oder durch die Auswertungseinrichtung und/oder durch den Arzt. Bei Unter- bzw. Überschreiten eines definierten Sollwertes bzw. eines sogenannten Diskriminationsfensters ist es erfindungsgemäß von Vorteil, wenn automatisch an dem Meß- und Übertragungsvorgang beteiligte Personen benachrichtigt werden. In diesem Zusammenhang bietet es sich an, die Auswertungseinrichtung in ein Netzwerk einzubinden, so daß die meßwertspezifische Information und/oder die Therapieempfehlung von dem Netzwerk abgerufen werden können.

Bei der Auswertungseinrichtung kann es sich beispielsweise um einen Server oder ein Dienstleistungs-Service-Zentrum handeln, wobei die meßwertspezifische Information an die Auswertungseinrichtung zum Beispiel in Form einer SMS übertragen wird. Ein Programm der Auswertungseinrichtung vergleicht die meßwertspezifischen Informationen mit vorgegebenen Sollwerten und/oder mit bereits erfaßten Werten. Daraus kann eine Therapieempfehlung abgeleitet werden, welche darüber entscheidet, ob eine sofortige medizinische Intervention erforderlich ist. Bei kritischen Zuständen der Körperfunktion kann die Therapieempfehlung auch automatisch an den Patienten und/oder behandelnden Arzt und/oder einen Rettungsdienst übertragen werden. Durch Einbindung der Auswertungseinrichtung in ein WAN oder ein LAN können Therapieempfehlungen bzw.meßwertspezifische Informationen an jedem Ort abgefragt werden. Somit ist es möglich, Körperfunktionswerte online zu überwachen und in Notfällen unmittelbar einzugreifen.

Bei dem erfindungsgemäßen Verfahren ist es außerdem von Vorteil, wenn an wenigstens einer Stelle des Meß- und/oder des Übertragungsvorganges eine Speichereinrichtung zur Speicherung des Meßwertes und/oder der meßwertspezifischen Information und/oder der Therapieempfehlung vorgesehen wird. Dadurch ist es möglich, bei einem möglichen Datenverlust während der Datenübertragung auf bereits vorhandene ältere Meßwerte zuzugreifen. Zudem wird durch die Speicherung der Daten der Vergleich vieler, über einen längeren Zeitraum aufgenommener Daten möglich. Somit können Hinweise auf den Erfolg einer bestimmten Therapie oder auch die Entwicklung der Körperfunktionen über einen vorgegebenen Zeitraum gezogen werden. Ferner ist es möglich, gemittelte und standardisierte Referenzwerte aus den Meßwerten zu bilden. Vorzugsweise sollte die Speichereinrichtung in der Auswertungseinheit und/oder in der mobilen Sende- und Empfangseinrichtung integriert sein. Weiterhin ist es erfindungsgemäß auch vorgesehen, daß die Speichereinrichtung in ein Netzwerk, insbesondere ein WAN eingebunden ist, so daß die gespeicherten Daten beispielsweise über das Internet abgerufen werden können.

Durch die Einbindung der Auswertungseinrichtung und/oder der Speichereinrichtung und/oder der übrigen an dem Verfahren beteiligten Einrichtungen in ein Netzwerk, insbesondere ein WAN, wobei diese Einbindung vorzugsweise über Funk erfolgt und die Informationen in einem Standardprotokoll eines Telekommunikationsdienstes übertragen werden, können eine beliebige Anzahl von Meßgeräten und mobilen Sende- und Empfangseinrichtungen miteinander und mit Auswertungseinrichtungen zusammenwirken, so daß in der Auswertungseinrichtung und/oder durch den behandelnden Arzt bzw. ein Krankenhaus oder dergleichen eine Vielzahl von Patientenmeßwerten gleichzeitig ausgewertet werden können.

Zur Sicherstellung der Anonymität des Patienten und der Sicherheit bei der Datenübertragung ist es außerdem erfindungsgemäß vorgesehen, den Meßwert und/oder die meßwertspezifische Information und/oder die Therapieempfehlung zu verschlüsseln. Dadurch wird einem beliebigen Dritten der Einblick in vertrauliche Patientendaten verwehrt. Die Verschlüsselung kann vorzugsweise vor der Übertragung der meßwertspezifischen Informationen in der mobilen Sende- und Empfangsstation bzw. vor der Übertragung der Therapieempfehlung in der Auswertungseinrichtung stattfinden. Darüber hinaus ist es erfindungsgemäß von Vorteil, vor der Datenspeicherung eine Datenverschlüsselung durchzuführen, wobei beispielsweise die Patientennamen, z.B. in Form von Kennummern, gespeichert werden.

Zur Steigerung der Übertragungsgeschwindigkeit oder auch zur Verringerung des Speicherbedarfs bietet es sich erfindungsgemäß an, an einer Stelle des Meß- und/oder des Übertragungsvorgangs eine Datenkompression vorzusehen, vorzugsweise vor der Übertragung der Daten und/oder vor der Datenspeicherung.

Die auf Grundlage der meßwertspezifischen Information in der Auswertungseinrichtung erstellte Therapieempfehlung kann auch unmittelbar von der Auswertungseinrichtung an das Meßgerät und/oder die mobile Sende- und Empfangseinrichtung übertragen werden und dort eine medizinische Intervention auslösen und/oder den Patienten über den aktuellen Stand der gemessenen Körperfunktion informieren. Dazu wird erfindungsgemäß die Therapieempfehlung ebenfalls drahtlos über das Mobilfunknetz in einem Standardprotokoll eines Telekommunikationsdienstes, vorzugsweise als elektronische Nachricht, übertragen.

Die Therapieempfehlung kann generell dazu genutzt werden, auf die Körperfunktion regulierend einzuwirken. Das kann entweder unmittelbar durch eine an dem Meßgerät vorgesehene Eingriffsfunktion erfolgen, bei der von dem Meßgerät beispielsweise ein Medikament entsprechend der Therapieempfehlung dosiert und dem Patienten zugeführt wird. Die Therapieempfehlung kann jedoch von der Auswertungseinrichtung auch mittelbar an die mobile Sende- und Empfangseinrichtung übertragen werden, wobei es dann dem Patienten überlassen ist, in die Körperfunktion regulierend einzugreifen oder entsprechende Verhaltensmaßregeln durchzuführen. Weiterhin ist es möglich, daß das mobile Sende- und Empfangsgerät eine entsprechenden Information an die Eingriffsfunktion des Meßgerätes aussendet.

Von Vorteil ist es in diesem Zusammenhang auch, wenn die Messung und/oder die Übertragung und/oder die Generierung des Meßwertes und/oder der meßwertspezifischen Information und/oder der Therapieempfehlung automatisch zeit- und/oder ergebnisgesteuert erfolgt. Diese Vorgehensweise entbindet den Patienten und den behandelnden Arzt von der regelmäßigen Kontrolle und von der Notwendigkeit, zu bestimmten Zeiten jeweils einen Meßvorgang aufzurufen und anschließend eine Therapieempfehlung durchzuführen. Selbstverständlich ist es jedoch auch möglich, daß die Messung und/oder die Übersendung und/oder die Generierung in definierten Zeitintervallen oder kontinuierlich erfolgt.

Um kritische Zustände der Körperfunktionen rechtzeitig erkennen zu können, bietet es sich an, bei Abweichung der Meßwertes von vorgegebenen Soll-Werten ein Warnsignal, vorzugsweise eine Sprachmitteilung, an die mobilen Sende- und Empfangseinheit und/oder das Meßgerät zu übermitteln. Das Warnsignal kann dabei automatisch ausgelöst werden und z.B. dazu dienen, einen Kontrollmessvorgang auszulösen. Natürlich ist es auch möglich, daß der behandelnde Arzt manuell nach entsprechender Kontrolle der meßwertspezifischen Informationen und/oder der Therapieempfehlung ein Warnsignal auslöst. Ebenso ist es möglich, daß der Patient von der mobilen Sende- und Empfangseinrichtung bei Erkennung einer körperlichen Fehlfunktion oder bei plötzlicher Veränderung seines Befindens ein Warnsignal an die Auswertungseinrichtung oder den behandelnden Arzt sendet, worauf automatisch und/oder manuell ein erneuter Meßvorgang und/oder eine medizinische Intervention erfolgt.

Das erfindungsgemäße Verfahren kann prinzipiell bei allen medizinischen bzw. sonstigen Meßgeräten eingesetzt werden, mit denen Körperfunktionen beliebiger Art gemessen werden können. Vorzugsweise können Blutzuckermeßwerte, die Körpertemperatur oder kardiologisch relevante Parameter gemessen werden.

Weitere bevorzugte Ausgestaltungen der vorliegenden Erfindung ergeben sich aus den Unteransprüchen.

Nachfolgend wird die Erfindung anhand eines Ausführungsbeispiels beschrieben. Dabei zeigt
- Fig. 1: eine schematische Darstellung des erfindungsgemäßen Verfahrens und des erfindungsgemäßen Systems mit wenigstens einer erfindungsgemäßen Vorrichtung und
- Fig. 2: eine schematische Darstellung einer Ausführungsform des Meßgerätes.

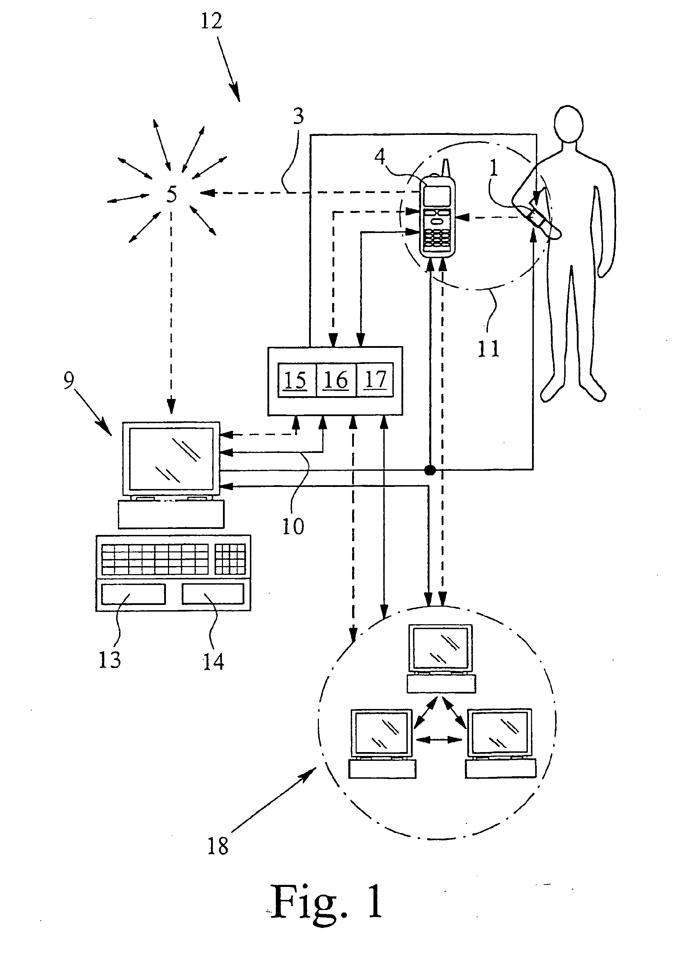

In Fig. 1 ist schematisch eine Variante des erfindungsgemäßen Verfahrens und eine Vorrichtung 11 zur Messung und Übertragung der Funktionswerte von Körperfunktionen dargestellt. Weiterhin ist in Fig. 1 schematisch ein System 12 mit wenigstens einer Vorrichtung 11 nach dem erfindungsgemäßen Verfahren dargestellt. Die Vorrichtung 11 weist ein Meßgerät 1 und eine mobile Sende- und Empfangseinrichtung 4 auf, wobei die mobile Sende- und Empfangseinrichtung 4 zur Übertragung einer meßwertspezifischen Information 3 als elektronische Nachricht in ein Mobilfunknetz 5 eingebunden ist. Die mobile Sende- und Empfangseinheit 4 überträgt die meßwertspezifische Information 3 an bzw. in das Mobilfunknetz 5, von wo aus die meßwertspezifische Information 3 an eine Auswertungseinrichtung 9 übertragen wird. Die Auswertungseinrichtung 9 kann unter anderem eine Auswertungsfunktion 13 zur Auswertung der meßwertspezifischen Information 3 und/oder weiterer Daten und/oder eine Generierungsfunktion 14 zur Generierung wenigstens einer Therapieempfehlung 10 zur medizinischen Intervention und/oder zur Information des Patienten bzw. des behandelnden Arztes aufweisen. Die von der Auswertungseinrichtung generierte Therapieempfehlung 10 kann ebenfalls drahtlos in umgekehrter Richtung an das Meßgerät 1 und/oder die mobile Sende- und Empfangseinrichtung 4 übertragen werden.

Nicht dargestellt ist, daß eine Therapieempfehlung auch auf der mobilen Sende- und Empfangseinheit 4 und/oder auf dem Meßgerät 1 selbst in Abhängigkeit von dem gemessenen Meßwert 2 generiert werden kann. Eine auf der mobilen Sende- und Empfangseinheit 4 und/oder auf dem Meßgerät 1 erstellte bzw. generierte Therapieempfehlung kann vorzugsweise zusammen mit einem Meßwert 2 als meßwertspezifische Information 3 in Form einer elektronischen Nachricht in das Mobilfunknetz 5 übertragen werden. Es ist im übrigen nicht dargestellt, daß die meßwertspezifische Information auch zwischen mehreren mobilen Sende- und Empfangseinrichtungen bzw. mehreren Auswertungseinrichtungen übertragen werden kann.

Des weiteren weist das System 12 wenigstens eine Speichereinrichtung 15, eine Verschlüsselungseinrichtung 16 und eine Kompressionseinrichtung 17 auf. Diese Einrichtungen können entweder integraler Bestandteil der mobilen Sende- und Empfangseinrichtung 4 und/oder der Auswertungseinrichtung 9 sein, sie können aber auch selbstständige Einrichtungen sein, die entsprechend in das System 12, das wenigstens eine Vorrichtung 11 aufweist und zur Durchführung des erfindungsgemäßen Verfahrens dient, eingebunden sein.

Die Speichereinrichtung 15 hat die Aufgabe, die meßwertspezifische Information 3 und/oder die Therapieempfehlung 10 zu speichern. Dabei ist es möglich, die gespeicherte meßwertspezifische Information 3 und die gespeicherte Therapieempfehlung 10 ausgehend von der Auswertungseinrichtung 9 abzurufen. Zudem ist es auch möglich, daß die gespeicherte meßwertspezifische Information 3 und/oder Therapieempfehlung 10 an ein Netzwerk 18 übertragen wird. Es ist jedoch auch möglich, Daten ausgehend von dem Netzwerk 18 an die Auswertungseinrichtung 9 und/oder die mobile Sende- und Empfangseinheit 4 zu übertragen. Dadurch ist gewährleistet, daß ein mit dem Netzwerk 18 verbundener Teilnehmer auf die meßwertspezifische Information 3 und/oder die Therapieempfehlung 10 zugreifen kann und seinerseits auch Therapieempfehlungen 10 und/oder meßwertspezifische Informationen 3 zurücksenden kann. Im Prinzip können bei dem erfindungsgemäßen Verfahren die meßwertspezifisch Information und/oder die Therapieempfehlung nahezu beliebig zwischen den bei dem Verfahren vorgesehenen Einrichtungen und den in das Netz eingebundenen Empfangs- und/oder Sendestationen bzw. den jeweiligen Teilnehmern ausgetauscht werden.

Im übrigen ist die Speicherung und/oder die Verschlüsselung und/oder die Kompression der meßwertspezifischen Information und/oder der Therapieempfehlung sowohl in der Auswertungseinrichtung 9 als auch an jeder beliebigen Stelle zwischen dem Meß- und dem Übertragungsvorgang möglich.

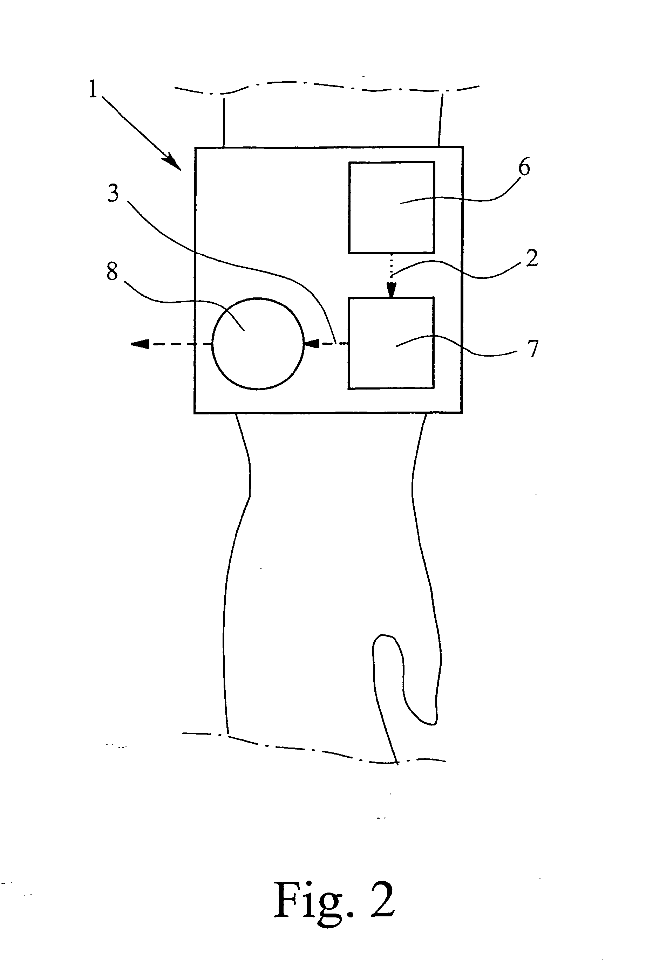

In der Fig. 2 wird nun der schematische Aufbau eines vorschlagsgemäßen Meßgerätes 1 dargestellt. Die Meßeinrichtung 6 dient zur Erfassung des Meßwertes 2. Der Meßwert 2 wird an einen Meßwertwandler 7 übergeben, wo der Meßwert 2 in eine meßwertspezifische Information 3 transformiert wird. Die meßwertspezifische Information 3 wird an die integrierte Sendeeinrichtung 8 des Meßgerätes 1 übertragen und von dort aus vorzugsweise drahtlos an die mobile Sende- und Empfangseinrichtung 4 übertragen.

Nicht dargestellt ist im übrigen, daß die integrierte Sendeeinrichtung 8 des Meßgerätes 1 ebenfalls eine Empfangseinrichtung aufweisen kann. Dadurch ist es möglich, daß Daten, die z.B. eine medizinische Intervention und/oder einen erneuten Meßvorgang auslösen, unmittelbar auf das Meßgerät 1 übertragen werden können. Das Meßgerät 1 selbst ist vorzugsweise an einer beliebigen Stelle des Körpers befestigt, wobei der Patient vorzugsweise so mit dem Meßgerät verbunden ist, daß jederzeit ein Meßvorgang möglich ist. Alternativ kann das Meßgerät 1 auch eigenständig in unmittelbarer Nähe des Körpers vorgesehen werden, wobei das Meßgerät transportabel ist. Damit kann der Patient beispielsweise auf Reisen das Meßgerät in einer Unterkunft hinterlegen und dort bei Bedarf Messungen durchführen und diese zur Auswertung per Mobilfunktelefon an den Arzt oder die Auswertungseinheit übertragen.

## Patentansprüche

1. System zur Messung und Übertragung von Körperfunktionswerten eines Patienten, insbesondere von Blutzuckerwerten, wobei von einem Messgerät (1) wenigstens ein Körperfunktionswert messbar bzw. ein Messwert (2) einer Körperfunktion aufnehmbar ist, wobei das Messgerät (1) ausgebildet ist, eine messwertspezifische Information (3) über eine drahtlose Schnittstelle an eine mobile Sende- und Empfangseinrichtung (4), vorzugsweise an ein Mobiltelefon, zu übertragen und wobei die mobile Sende- und Empfangseinrichtung (4) dazu ausgebildet ist, die messwertspezifische Information (3) in Form einer elektronischen Nachricht eines Telekommunikationsdienstes in einem Standardprotokoll in ein Mobilfunknetz (5) zu übertragen und wobei die messwertspezifische Information (3) von der mobilen Sende- und Empfangseinrichtung (4) über das Mobilfunknetz (5) an wenigstens eine Auswertungseinrichtung (9) übertragbar ist, die einen Soll-Istwert-Vergleich des Messwertes vornimmt,
**dadurch gekennzeichnet,**
**dass** die Auswertungseinrichtung ausgebildet ist, in Abhängigkeit von dem Messwert (2) eine Therapieempfehlung (10) zur Therapierung eines Fehlverhaltens der Körperfunktion zu generieren, wobei zur Generierung der Therapieempfehlung (10) der Sollwert-Istwert-Vergleich des Messwertes (2) erfolgt und wobei die Therapieempfehlung (10) automatisch bei Messung pathologischer Körperfunktionswerte erstellt wird, und wobei die Therapieempfehlung (10) dazu ausgebildet ist, eine Medikamentendosierung zu ermöglichen.

2. System nach Anspruch 1, **dadurch gekennzeichnet, dass** die messwertspezifische Information (3) als SMS (Short-Message-Service) oder als EMS (Enhanced Message Service) oder als MMS (Multimedia Message Service) oder als email in ein nach dem GSM- oder DCS- oder GPRS- oder UMTS-Standard arbeitendes Mobilfunknetz übertragbar ist.

3. System nach Anspruch 1 oder 2, **dadurch gekennzeichnet, dass** das System dazu ausgebildet ist, dass der Messwert (2) in wenigstens einer Messeinrichtung (6) des Messgerätes (1) aufgenommen bzw. gemessen wird, wobei der aufgenommene bzw. gemessene Messwert (2) über wenigstens einen Messwertwandler (7) in die messwertspezifische Information (3) transformiert wird und dass die messwertspezifische Information (3) über wenigstens eine integrierte Sendeeinrichtung (8) des Messgerätes (1) an die mobile Sende- und Empfangseinrichtung (4) übertragen wird, vorzugsweise über eine Blue-Tooth-Funkverbindung.

4. System nach einem der vorhergehenden Ansprüche, **dadurch gekennzeichnet, dass** das System dazu ausgebildet ist, dass als messwertspezifische Information wenigstens ein dem Messwert entsprechender Zahlenwert übertragen wird.

5. System nach einem der vorhergehenden Ansprüche, **dadurch gekennzeichnet, dass** das System dazu ausgebildet ist, dass die zu übertragende messwertspezifische Information (3) als elektronische Nachricht auf der mobilen Sende- und Empfangseinrichtung (4) angezeigt wird.

6. System nach einem der vorhergehenden Ansprüche, **dadurch gekennzeichnet, dass** das System dazu ausgebildet ist, dass die messwertspezifische Information (3) in ein Netzwerk, insbesondere ein WAN übertragen wird.

7. System nach einem der vorhergehenden Ansprüche, **dadurch gekennzeichnet, dass** das System dazu ausgebildet ist, dass an wenigstens einer Stelle des Mess- und/oder des Übertragungsvorgangs der Messwert (2) und/oder die messwertspezifische Information (3) gespeichert und/oder verschlüsselt wird und/oder dass an wenigstens einer Stelle des Mess- und/oder des Übertragungsvorgangs eine Datenkompression vorgenommen wird und/oder dass eine gebündelte Nachrichtenübertragung vorgesehen wird.

8. System nach einem der vorhergehenden Ansprüche, **dadurch gekennzeichnet, dass** das System dazu ausgebildet ist, dass die Messung des Messwertes (2) und/oder die Übertragung der messwertspezifischen Information (3) und/oder die Generierung der Therapieempfehlung (10) automatisch und/oder zeit- und/oder ergebnisgesteuert und/oder in definierten Zeitintervallen und/oder kontinuierlich erfolgt.

9. System nach einem der vorherigen Ansprüche, **dadurch gekennzeichnet, dass** das System dazu ausgebildet ist, dass bei Abweichung des Messwertes (2) von wenigstens einem vorgegebenen Sollwert ein Warnsignal, vorzugsweise eine Sprachmitteilung, an die mobile Sende- und Empfangseinheit (4) und/oder das Messgerät (1) und/oder an die Auswerteeinrichtung (9) übermittelt wird.

10. System nach einem der vorhergehenden Ansprüche, **dadurch gekennzeichnet, dass** das System dazu ausgebildet ist, dass als Messwert (2) ein Blutzuckermesswert und/oder die Körpertemperatur und/oder ein kardiologisch relevanter Parameter gemessen wird.

## Claims

1. A system for measuring and transmitting functional values of a patient's body, especially of blood sugar values, wherein at least one functional value of the body can be measured by a measuring instrument (1) or a measured value (2) of a body function can be acquired, wherein the measuring instrument (1) is designed to transmit information (3) specific to the measured value via a wireless interface to a mobile transmitter and receiver device (4), preferably to a mobile phone, and wherein the mobile transmitter and receiver device (4) is designed to transmit the information (3) specific to the measured value in the form of an electronic message of a telecommunication service in a standard protocol to a mobile radio network (5) and wherein the information (3) specific to the measured value can be transmitted by the mobile transmitter and receiver device (4) via the mobile radio network (5) to at least one evaluation device (9), which makes a target-actual value comparison of the measured value,
**characterized in**
**that** the evaluation device is designed, depending on the measured value (2) to generate a therapy recommendation (10) for the treatment of a malfunction of the body function, wherein to generate the therapy recommendation (10) the target value-actual value comparison of the measured value (2) is made and wherein the therapy recommendation (10) is automatically created with the measurement of the pathological functional value of the body, and wherein the therapy recommendation (10) is designed to permit a medication dosage.

2. A system according to claim 1, **characterized in that** the information (3) specific to the measured value can be transmitted as SMS (Short-Message-Service) or as EMS (Enhanced Message Service) or as MMS (Multimedia Message Service) or as an e-mail in a mobile radio network operating according to the SMS- or DCS- or GPRS- or UMTS-standard.

3. A system according to claim 1 or 2, **characterized in that** the system is designed, so that the measured value (2) is acquired or measured in at least one measuring device (6) of the measuring instrument (1), wherein the acquired or measured measured value (2) is transformed via at least one transducer (7) into the information (3) specific to the measured value and that the information (3) specific to the measured value is transmitted via at least one integrated transmitter device (8) of the measuring instrument (1) to the mobile transmitter and receiver device (4), preferably via a Bluetooth radio connection.

4. A system according to one of the preceding claims, **characterized in that** the system is designed so that at least one numerical value corresponding to the measured value is transmitted as information specific to the measured value.

5. A system according to one of the preceding claims, **characterized in that** the system is designed so that the information (3) specific to the measured value to be transmitted is displayed as an electronic message on the mobile transmitter and receiver device (4).

6. A system according to one of the preceding claims, **characterized in that** the system is designed so that the information (3) specific to the measured value is transmitted to a network, in particular to a WAN.

7. A system according to one of the preceding claims, **characterized in that** the system is designed so that at at least one point of the measuring and/or of the transmission process the measured value (2) and/or the information (3) specific to the measured value is saved and/or is encrypted and/or that at least at one point of the measuring and/or of the transmission process a data compression is undertaken and/or that a bundled message transmission is provided.

8. A system according to one of the preceding claims, **characterized in that** the system is designed so that the measurement of the measured value (2) and/or the transmission of the information (3) specific to the measured value and/or the generation of the therapy recommendation (10) occurs automatically and/or time-controlled and/or results-oriented and/or in the defined time intervals and/or continuously.

9. A system according to one of the preceding claims, **characterized in that** the system is designed so that in the case of the deviation of the measured value (2) from at least one predetermined target value a warning signal, preferably a voice mail, is transmitted to the mobile transmitter and receiver unit (4) and/or to the measuring instrument (1) and/or to the evaluation device (9).

10. A system according to one of the preceding claims, **characterized in that** the system is designed so that a blood sugar measured value and/or the body temperature and/or a cardiologically-relevant parameter is measured as a measured value (2).

## Revendications

1. Système destiné à la mesure et à la transmission de valeurs de fonctions corporelles d'un patient, en particulier de valeurs de glycémie, dans lequel au moins une valeur de fonction corporelle peut être mesurée ou une valeur de mesure (2) d'une fonction corporelle peut être détectée par un appareil de mesure (1), dans lequel l'appareil de mesure (1) est conçu pour transmettre une information spécifique à la valeur de mesure (3) à un dispositif d'émission et de réception mobile (4), de préférence un téléphone portable, par le biais d'une interface sans fil et dans lequel le dispositif d'émission et de réception mobile (4) est conçu pour transmettre l'information spécifique à la valeur de mesure (3) sous la forme d'un message électronique d'un service de télécommunication dans un protocole standard dans un réseau de téléphonie mobile (5), l'information spécifique à la valeur de mesure (3) pouvant être transmise à au moins un dispositif d'analyse (9), qui effectue une comparaison entre la valeur de consigne et la valeur effective de la valeur de mesure,
**caractérisé**
**en ce que** le dispositif d'analyse est conçu pour générer, en fonction de la valeur de mesure (2), une recommandation thérapeutique (10) pour le traitement d'une déficience de la fonction corporelle, la comparaison entre la valeur de consigne et la valeur effective de la valeur de mesure (2) étant réalisée afin de générer la recommandation thérapeutique (10) et la recommandation thérapeutique (10) étant créée automatiquement en cas de mesure de valeurs de fonctions corporelles pathologiques, et la recommandation thérapeutique (10) étant conçue pour permettre le dosage d'un médicament.

2. Système selon la revendication 1, **caractérisé en ce que** l'information spécifique à la valeur de mesure (3) peut être transmise en tant que SMS (Short Message Service) ou en tant qu'EMS (Enhanced Message Service) ou en tant que MMS (Multimedia Message Service) ou en tant qu'e-mail dans un réseau de téléphonie mobile fonctionnant conformément à la norme GSM ou DCS ou GPRS ou UMTS.

3. Système selon la revendication 1 ou 2, **caractérisé en ce que** le système est conçu de telle sorte que la valeur de mesure (2) est détectée ou mesurée dans au moins un dispositif de mesure (6) de l'appareil de mesure (1), la valeur de mesure détectée ou mesurée (2) étant transformée dans l'information spécifique à la valeur de mesure (3) par le biais d'au moins un convertisseur de valeur de mesure (7), et **en ce que** l'information spécifique à la valeur de mesure (3) est transmise au dispositif d'émission et de réception mobile (4) par le biais d'au moins un dispositif d'émission intégré (8) de l'appareil de mesure (1), de préférence par le biais d'une liaison radio Blue Tooth.

4. Système selon l'une des revendications précédentes, **caractérisé en ce que** le système est conçu de telle sorte qu'au moins une valeur chiffrée correspondant à la valeur de mesure est transmise en tant qu'information spécifique à la valeur de mesure.

5. Système selon l'une des revendications précédentes, **caractérisé en ce que** le système est conçu de telle sorte que l'information spécifique à la valeur de mesure (3) qui doit être transmise est affichée en tant que message électronique sur le dispositif d'émission et de réception mobile (4).

6. Système selon l'une des revendications précédentes, **caractérisé en ce que** le système est conçu de telle sorte que l'information spécifique à la valeur de mesure (3) est transmise dans un réseau, en particulier un WAN.

7. Système selon l'une des revendications précédentes, **caractérisé en ce que** le système est conçu de telle sorte qu'à au moins un stade de l'opération de mesure et/ou de transmission, la valeur de mesure (2) et/ou l'information spécifique à la valeur de mesure (3) est enregistré(e) et/ou crypté(e) et/ou **en ce qu'**à au moins un stade de l'opération de mesure et/ou de transmission, une compression de données est réalisée et/ou **en ce qu'**une transmission de messages par paquets est prévue.

8. Système selon l'une des revendications précédentes, **caractérisé en ce que** le système est conçu de telle sorte que la mesure de la valeur de mesure (2) et/ou la transmission de l'information spécifique à la valeur de mesure (3) et/ou la génération de la recommandation thérapeutique (10) a ou ont lieu automatiquement et/ou en fonction du temps et/ou des résultats et/ou à des intervalles de temps définis et/ou de façon continue.

9. Système selon l'une des revendications précédentes, **caractérisé en ce que** le système est conçu de telle sorte que si la valeur de mesure (2) dévie d'au moins une valeur de consigne prédéfinie, un signal d'avertissement, de préférence un message vocal, est transmis à l'unité d'émission et de réception mobile (4) et/ou à l'appareil de mesure (1) et/ou au dispositif d'analyse (9).

10. Système selon l'une des revendications précédentes, **caractérisé en ce que** le système est conçu de telle sorte qu'une valeur de mesure de la glycémie et/ou la température corporelle et/ou un paramètre pertinent sur le plan cardiologique est mesuré(e) en tant que valeur de mesure (2).
